# EUROPÄISCHE PATENTSCHRIFT

(11) **EP 1 038 544 B1**
(45) Veröffentlichungstag und Bekanntmachung des Hinweises auf die Patenterteilung: **07.06.2006**
(21) Anmeldenummer: 00105664.7
(22) Anmeldetag: 17.03.2000
(51) Int. Cl.: A61M 15/00

(54) **Vorrichtung zur Flussbegrenzung bei niedrigen Differenzdrücken**
Apparatus to limit the flow at low differential pressures
Appareil de limiter l'écoulement à faible pressions différentielles

(30) Priorität: 19.03.1999 DE 19912461
(43) Veröffentlichungstag der Anmeldung: 27.09.2000
(73) Patentinhaber: GSF - Forschungszentrum für Umwelt und Gesundheit GmbH, 85764 Oberschleissheim (DE)
(72) Erfinder: Müllinger, Bernhard, 84339 Huldsessen (DE); Scheuch, Gerhard, 35285 Gmuenden (DE); Sommerer, Knut, 81241 München (DE); Haas, Friedel, 82131 Gauting (DE); Roeder, Sascha, 85053 Ingolstadt (DE)
(74) Vertreter: Reinhard - Skuhra - Weise & Partner

(56) Entgegenhaltungen:
- EP-A- 0 341 573
- WO-A-84/01293
- DE-A- 19 504 750
- US-A- 4 284 505
- US-A- 4 350 477
- US-A- 4 573 640
- US-A- 4 756 508
- US-A- 5 858 569

## Beschreibung

Die Erfindung betrifft eine Vorrichtung zur Flussbegrenzung bei niedrigen Differenzdrücken, insbesondere für das Begrenzen des Inhalationsvolumenstroms bei der Inhalation von therapeutischen Aerosolen, gemäß der Oberbegriffe der Ansprüche 1, 15 und 19.

Im Bereich der Aerosoltherapie ist es für die vorgesehene Verabreichung des Medikaments wichtig, dass zum einen ein bestimmter Inhalationsvolumenstrom nicht überschritten wird und andererseits nur ein möglichst geringer Druckabfall bei Volumenströmen unterhalb des maximalen Volumenstroms auftritt. Diesen Bedingungen genügen die bisher bekannten Flussbegrenzungsvorrichtungen nicht, da sie meistens nur bei Drücken weit über 100 mbar Überdruck arbeiten.

Eine Vorrichtung der eingangs genannten Gattung ist bereits in der WO84/01293 A offenbart. Diese vorbekannte Anordnung dient dazu, die für eine Sauerstofftherapie benötigte Sauerstoffmenge mit dem Ziel zu minimieren, einen vorgegebenen oder gewünschten Sauerstoffpegel in dem Blut unter Berücksichtigung von ausgeatmeter Luft beizubehalten. Hierzu ist eine hohle Vorrichtung vorgesehen, die an gegenüberliegenden Enden Einlassöffnungen für das Zuführen von Sauerstoff unter Druck sowie zwei in der Mitte der Vorrichtung vorgesehene Auslassöffnungen zum Einsatz in die Nase aufweist, wobei der Hohlkörper über dem Bereich zwischen den Einlassöffnungen und den Auslassöffnungen eine elastische Membran aufweist. Die elastische Membran ist nach Beginn des Inhalierens aufgrund des entnommenen Volumens sowie aufgrund eines dadurch erzeugten teilweisen Vakuums bestrebt, sich in Richtung auf die gegenüberliegende Seite derart zu bewegen, dass die Sauerstoffströmung von den beiden Einlässen durch den Hohlkörper kurzzeitig verzögert wird. Die Membran dient dabei dazu, die aus den Einlassöffnungen seitlich in den Hohlkörper eintretenden Sauerstoffströme zu den dazwischenliegenden parallelen Auslassöffnungen 32 so zu verändern, dass eine ausreichende Sauerstoffbevorratung in dem Hohlkörper für eine Durchmischung mit der ausgeatmeten Luft, die durch die Auslassöffnung 32 in den Hohlkörper eintritt, zu erreichen. Der Hohlkörper ist dabei mit zwei Ballonabschnitten mit den Einlassöffnungen und einem mittleren rohrförmigen Abschnitt gebildet, wobei die Membran in der Ausgangsstellung im Bereich der Mittellängsachse des Hohlkörpers angeordnet ist. Der Bereich zwischen Einlassöffnung und Auslassöffnung ist dem gemäß mit einem Quergebilde, der sich aus einer kreisförmigen Innenwandung und einer im Trennfugenbereich des Hohlkörpers gehaltenen Membranwand mit exzellenter Gestaltung gebildet. Als nachteilig ist dem gemäß bei dieser vorbekannten Vorrichtung anzusehen, dass aufgrund des dort vorgesehenen Doppelkörperkonzepts mit einer Membran in Folge der getroffenen Konfiguration des Krümmungsbereichs keine ausreichende Empfindlichkeit erzielbar ist, die für eine Begrenzung des Inhalationsvolumens bei der Inhalation von therapeutischen Aerosolen in der Größenordnung von 5 mbar einsetzt.

Aus der US-A-4 573 640 ist eine Bewässerungseinrichtung bekannt, die einen langgestreckten flachen Körper mit einem querlaufenden Grundteil aufweist, unter dem eine im Wesentlichen längliche hohlraumähnliche Ausnehmung gebildet ist, die mit einer rechtwinkligen flexiblen Membran versehen ist, welche sich unter viel Druck unter eine untere Fläche des Grundteils zur Begrenzung einer Strömung durch eine Bohrung in diesen verformt.

In der DE 19 504 750 A ist ein Druckaugleichsgefäß offenbart, dass zwei sich gegenüberliegende Flanchanschlüsse besitzt, zwischen denen ein Membranrohr gelagert ist. Dieses Membranrohr dehnt sich bei Überdruck aus.

Der Erfindung liegt dem gemäß die Aufgabe zugrunde, eine Vorrichtung der eingangs genannten Gattung verfügbar zu machen, bei der bereits bei Drücken von 5 mbar eine selbsttätige mechanische Volumenstromregelung einsetzt.

Erfindungsgemäß wird diese Aufgabe durch die in den Patentansprüchen 1 bzw. 15 bzw. 19 genannten Merkmale gelöst.

Bevorzugte Merkmale, die die Erfindung vorteilhaft weiterbilden, sind den jeweils nachgeordneten Patentansprüchen zu entnehmen.

Aufgrund des erfindungsgemäßen Konzepts ist es vorteilhaft möglich, eine Vorrichtung zur Begrenzung des Inhalationsvolumens bei der Inhalation von therapeutischen Aerosolen verfügbar zu machen, bei der bereits bei Drücken von 5 mbar eine selbsttätige mechanische Volumenstromregelung einsetzt. Dabei lässt sich vorteilhaft durch die Wahl der Materialflexibilität bzw. -elastizität für den bzw. die Wandabschnitte jedes Strömungsbereichs bzw. -kanals, der bzw. die sich zur Verengung bzw. Einschnürung des Kanalquerschnitts abhängig vom Unterdruck im Strömungskanal nach innen durchbiegen, mit außerordentlich geringem technischen Aufwand eine Anpassung an verschiedene maximale Volumenströme erreichen. Wenn zudem das Material, mit dem die Vorrichtung aufgebaut ist, aus einem bioverträglichen Kunststoff besteht, insbesondere jeder Strömungskanal aus Silikon aufgebaut ist, ist eine hervorragende Eignung für den klinischen Einsatz gegeben.

Jeder Strömungskanal ist nach einer bevorzugten Ausgestaltung mit einem flachen länglichen Querschnitt ausgebildet, wobei dieser Querschnitt mit gegenüberliegenden großflächigen Wänden gebildet ist. Hierdurch wird eine Durchbiegung der Wände nach innen zur Verringerung des Querschnitts des Kanals begünstigt.

Gemäß einer bevorzugten weiteren Ausgestaltung der Erfindung sind die gegenüberliegenden Wände jedes Strömungskanals an ihrer Außenseite wenigstens im Bereich der Mitte zwischen Einsaug- und Aussaugöffnung zur Umgebung offen, wobei bevorzugt jede Wand an ihrer Außenseite wenigstens im Bereich der Mitte zwischen Einsaug- und Aussaugöffnung einen Kammerabschnitt aufweist, der über eine Bohrung zur Umgebung geöffnet ist. Durch diese konstruktiven Maßnahmen ist vorteilhaft für den nötigen Druckausgleich beim Zusammenziehen der Wände gesorgt.

In konstruktiv einfacher Weise ist weiterhin vorgesehen, dass jeder Strömungskanal einen schichtweisen Aufbau aufweist, vorzugsweise aus einer geschlossenen Wand, einer gleich großen rahmenförmigen Zwischenwand und einer gleich großen Wand mit Einsaug- und Aussaugöffnung, wobei die gegenüberliegenden Wände randseitig in dem Gehäuse befestigt sind.

Als Material für die Ausgestaltung jedes Strömungskanals ist jedes flexible und bioverträgliche Material geeignet, das flexibel und nach dem Verbiegen auch rückverformbar ist. Vorzugsweise bestehen wenigstens die großflächigen Kanalwände, bevorzugt auch die Zwischenwand, aus Silikonmatten, und das Gehäuse aus einem vorzugsweise bioverträglichen Kunststoff.

Nach einer besonderen Ausgestaltung der Erfindung sind die Materialschichten für jeden Strömungskanal zwischen zwei Gehäuseabschnitten austauschbar befestigt. Hierdurch ist es in einfacher Weise möglich, eine Vorrichtung für verschiedene Durchflussbegrenzungsgrößen mit entsprechend zugeordnetem Strömungskanal zu verwenden. Dabei ist die Wanddicke der großflächigen Kanalwände bevorzugt jeweils gleich stark.

Gemäß einer alternativen weiteren Ausgestaltung der Erfindung ist vorteilhaft vorgesehen, dass statt eines schichtweisen Aufbaus ein einstückiger Aufbau jedes Strömungskanals, vorzugsweise in Form eines Silikonbauteils, vorgesehen ist.

Für eine Flussbegrenzung unabhängig vom Umgehungsdruck ist nach einer weiteren Ausgestaltung der Erfindung vorgesehen, dass jede Wand an ihrer Außenseite wenigstens im Bereich der Mitte zwischen der Ansaug- und der Aussaugöffnung einen Kammerabschnitt mit einer Bohrung aufweist, wobei die Bohrungen über einen Kanal bzw. einen Schlauch mit der Einsaugöffnung verbunden sind. Hierdurch wird der für die Regelung entscheidende Differenzdruck zwischen der Ansaug- und der Aussaugöffnung gemessen, und die Flussbegrenzung könnte auch in einem geschlossenen System betrieben werden.

Jeder Strömungskanal kann nach einer alternativen Ausgestaltung der Erfindung statt eines flachen länglichen Querschnitts auch einen ringförmigen Querschnitt aufweisen, wobei jeder Strömungskanal vorzugsweise in einem zylinderförmigen Gehäuse symmetrisch und von der Zylinderinnenwand beabstandet zwischen radialen Scheiben angeordnet ist. Diese Haltescheiben besitzen vorzugsweise ringsegmentförmige Einsaug- und Aussaugöffnungen, wobei die Haltescheibe mit den Einsaugöffnungen Belüftungsbohrungen für den zylinderförmigen Innenbereich und den ringförmigen Umgebungsbereich jedes Strömungskanals aufweist. Jeder ringförmige Strömungskanal ist bevorzugt mit Silikonmatten gebildet.

Gemäß einer weiteren alternativen Ausgestaltung der Erfindung ist vorgesehen, dass der Strömungsbereich zwischen einer zentralen Einsaugöffnung und diese radial umgebenden Aussaugöffnungen gebildet ist und sternförmige bzw. radiale Stege aufweist, die sich von einer gemeinsamen Bodenfläche zu der flexiblen Wand erstrecken und einschnürbare Strömungskanäle bilden. Hierdurch lässt sich die Vorrichtung außerordentlich kompakt gestalten, einfach herstellen und austauschen.

Die die Strömungskanäle bildenden Stege können unterschiedliche Länge aufweisen, um im Bereich der längeren Stege einen breiteren Strömungskanal zu bilden, der sich dann bei zwischengeordneten kürzeren Stegen in mehrere Strömungskanäle aufteilt. Der Querschnitt der Stege kann in radialer Richtung konstant sein. Vorteilhaft erweitern sich die Stege in ihrer Breite nach außen, wobei vorzugsweise zwischen zwei benachbarten Stegen eine Ansaugöffnung vorgesehen ist.

Ausgestalten lässt sich die Vorrichtung in einer für die Massenproduktion geeigneten Einweglösung vorteilhaft durch ein scheibenförmiges Gehäuse, in dem einstückig zwischen flachen Ausnehmungen die Stege und in den Ausnehmungen randseitig die Aussaugöffnungen gebildet sind, sowie durch eine auf den Stegen aufliegende dünne flexible Wand mit mittiger Einsaugöffnung, die im Randbereich des Gehäuses befestigt ist. Die Wand kann dabei im Randbereich des Gehäuses festgeklebt bzw. festgeschweißt oder mittels eines ringförmigen Montageteils festgeklemmt sein.

Die dünne flexible Wand besteht bevorzugt aus Silikon, Silikonkautschuk, Viton, Latex, Naturkautschuk oder einem anderen Elastomer.

Nachfolgend werden Ausführungsbeispiele der Erfindung schematisiert und anhand der beigefiigten Zeichnungen näher erläutert. Es zeigen:
- Fig. 1: eine Draufsicht auf eine Vorrichtung zur Begrenzung des Inhalationsvolumenstroms bei der Inhalation von therapeutischen Aerosolen;

- Fig. 2: eine Seitenansicht auf die in Fig. 1 gezeigte Vorrichtung;
- Fig. 3: einen Schnitt entlang der Schnittlinie III-III in Fig. 1;
- Fig. 4: einen Schnitt entlang der Schnittlinie IV-IV in Fig. 1;
- Fig. 5: einen Längsschnitt durch ein weiteres Ausführungsbeispiel einer Vorrichtung zur Begrenzung des Inhalationsvolumens bei der Inhalation von therapeutischen Aerosolen;
- Fig. 6: eine Unteransicht auf die in Fig. 5 gezeigte Vorrichtung;
- Fig. 7: eine Draufsicht auf ein weiteres Ausführungsbeispiel einer erfindungsgemäßen Vorrichtung zur Flussbegrenzung;
- Fig. 8: einen Schnitt entlang der Schnittlinie VIII-VIII in Fig. 7;
- Fig. 9: eine Draufsicht auf eine gegenüber Fig. 7 modifizierte Vorrichtung mit unterschiedlichen Steglängen; und
- Fig. 10: einen Schnitt entlang der Schnittlinie X-X in Fig. 9.

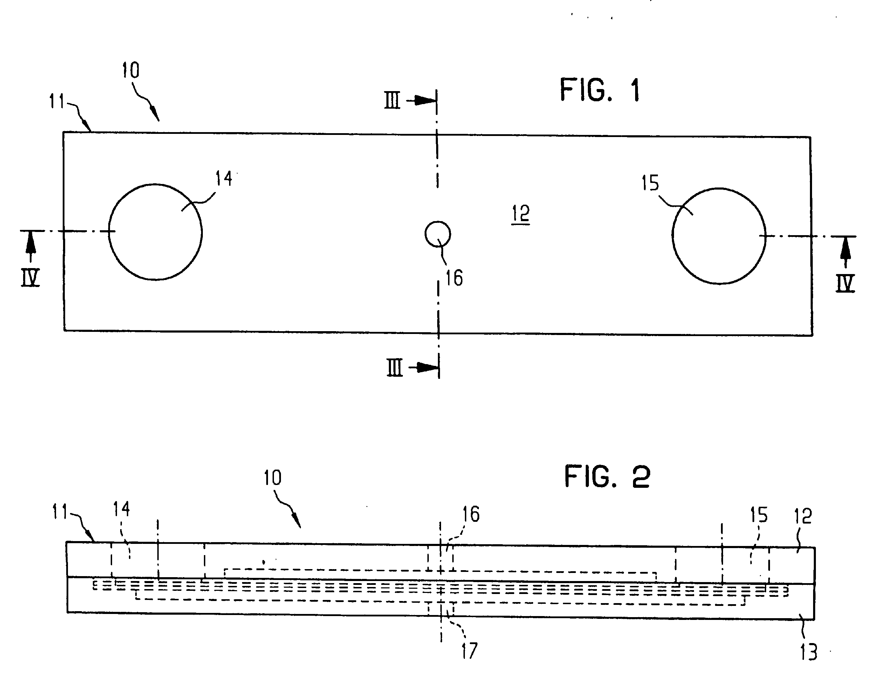

In Fig. 1 ist ein Ausführungsbeispiel einer Vorrichtung 10 zur Durchflussbegrenzung bei niedrigen Differenzdrücken, insbesondere zur Begrenzung des Inhalationsvolumenstroms, bei der Inhalation von therapeutischen Aerosolen dargestellt. Die Vorrichtung besteht aus einem langgestreckten quaderförmigen Gehäuse 11, das mit einer oberen plattenförmigen Gehäusehälfte 12 und einer unteren plattenförmigen Gehäusehälfte 13 gebildet ist. Das Gehäuse 11 besteht aus Kunststoff.

In das obere plattenförmige Gehäuseteil 12 sind eine Einsaugöffnung 14, eine Aussaugöffnung 15 sowie eine Belüftungsbohrung 16 eingebracht. Bei der unteren plattenförmigen Gehäusehälfte 13 ist genau fluchtend mit der Belüftungsbohrung 16 eine Belüftungsöffnung 17 vorgesehen.

Zwischen den plattenförmigen oberen und unteren Gehäusehälften 12 und 13 sind eine obere Wand (Silikonmatte) 18, eine Rahmensilikonmatte19 und eine untere Wand (Silikonmatte) 20 in nicht dargestellter Weise befestigt, beispielsweise durch randseitige Verschraubung der beiden plattenförmigen Gehäuseteile 12 und 13.

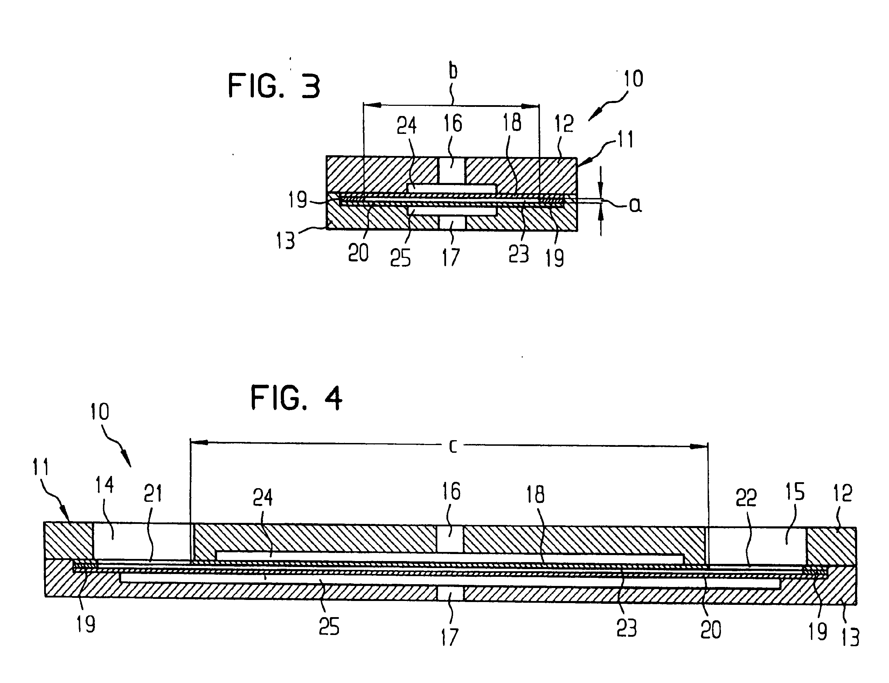

Die obere Wand 18 besitzt, wie aus Fig. 4 ersichtlich, eine mit der Einsaugöffnung 14 fluchtende Öffnung 21 sowie eine mit der Aussaugöffnung 15 fluchtende Öffnung 22. Wie sich im Zusammenhang mit den Fig. 3 und 4 ergibt, ist zwischen den Wänden 18 und 20 ein Strömungskanal 23 gebildet, während an der Innenseite der oberen plattenförmigen Gehäusehälfte 12 eine kammerförmige Ausnehmung in Form eines Kammerabschnitts 24 vorgesehen ist, der über die Belüftungsbohrung 16 zur Außenseite offen ist. An der Außenseite der unteren Wand 20 ist in der unteren plattenförmigen Gehäusehälfte 13 eine kammerförmige Ausnehmung in Form eines Kammerabschnitts 25 gebildet, der über die Belüftungsbohrung 17 zur Außenseite frei liegt und die sich seitlich bis in den Bereich der Öffnungen 21 bzw. 22 erstreckt. Der Kammerabschnitt 25 hat, wie aus dem Schnitt von Fig. 3 ersichtlich, dieselbe Breite wie der Kammerabschnitt 24, erstreckt sich jedoch jeweils in Längsrichtung über den parallel verlaufende Kammerabschnitt 24.

Der Strömungskanal 23 hat, wie aus den Fig. 3 und 4 ersichtlich, einen rechteckigen Querschnitt mit einer großen Breite b im Vergleich zu einer schmalen Höhe a, die der Materialstärke einer Zwischenwand 19 entspricht. Die Länge c des Strömungskanals 23 ist in Fig. 4 angegeben.

Wird durch die Aussaugöffnung 15 Luft in die Einsaugöffnung 14 eingesaugt, entsteht aufgrund des Strömungswiderstandes im Strömungskanal 23 ein Unterdruck. Dieser Unterdruck im Strömungskanal 23 sorgt dafür, dass sich die beiden Wände 18 und 20 nach innen durchbiegen und damit den Querschnitt des Strömungskanals 23 verengen. Diese Durchbiegung der Wände 18 und 20 ist umso stärker, je größer der Unterdruck im Strömungskanal 23 ist. Der Querschnitt des Strömungskanals 23 verändert sich somit in Abhängigkeit des Differenzdrucks zwischen Aussaugöffnung 15 und Einsaugöffnung 14, und da der Volumenstrom wiederum vom Querschnitt abhängt, erfolgt über die Querschnittsänderung eine direkte Regelung des Volumenstroms.

Durch die degressive Materialflexibilität nimmt dabei die für die Durchbiegung der Wände 18 und 20 erforderliche Kraft mit zunehmendem Unterdruck im Strömungskanal 23 bis zu einem Grenzwert zu, der den gewünschten minimalen Strömungskanalquerschnitt zur Begrenzung des Volumenstroms bestimmt.

Aufgrund des erfinderischen Konzepts bildet die erfindungsgemäße Vorrichtung somit ein Durchflussregelventil, das bereits bei Drücken von 5 mbar mechanisch den Volumenstrom regelt. Dieses Durchflussregelventil kann demzufolge in vorteilhafter Weise zur Begrenzung des Inhalationsvolumens in der Aerosoltherapie eingesetzt werden. Die Vorrichtung ist sowohl funktionell als auch vom technischen Aufwand her anderen bekannten Durchflussregelsystemen überlegen und lässt sich auch universell einsetzen und individuell an die jeweiligen Volumenstrom-Begrenzungsanforderungen durch entsprechende Materialwahl und/oder Dimensionierung der Einlegermatten bzw. Wände 18-20 anpassen. Die Vorrichtung eignet sich selbstverständlich nicht nur für den Bereich der Aerosoltherapie, sondern lässt sich in allen technischen Bereichen dort einsetzen, wo bei niedrigen Differenzdrücken eine Flussbegrenzung erwünscht wird.

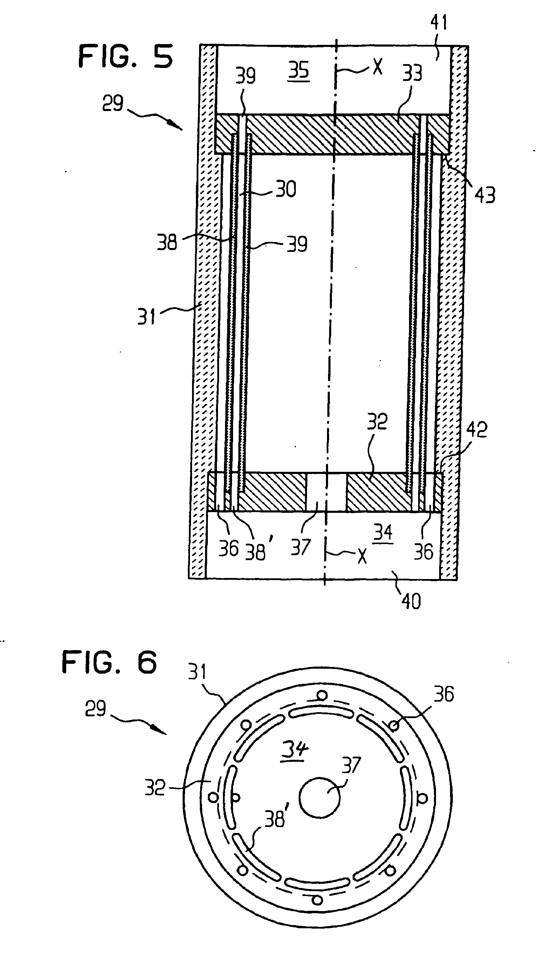

In Fig. 5 ist ein weiteres Ausführungsbeispiel einer Vorrichtung 29 gemäß der Erfindung dargestellt. Die Vorrichtung 29 besitzt ein zylinderförmiges Gehäuse 31, in dem ein Strömungskanal 30 mit einem ringförmigen Querschnitt von der Zylinderinnenwand beabstandet zwischen radialen Haltescheiben 32 und 33 angeordnet ist. Mit 34 ist die Einsaugöffnung an dem Gehäuse 31 und mit 35 die Aussaugöffnung an dem Gehäuse 31 bezeichnet. Der Strömungskanal 30 ist mit zylinderförmigen Silikonmatten 38 und 39 gebildet, die in den radialen Haltescheiben 32 und 33 eingesetzt sind.

Die der Einsaugöffnung 34 zugewandte Haltescheibe 32 besitzt für den ringförmigen Umgebungsbereich des Strömungskanals 30 Belüftungsbohrungen 36, die mit gleichmäßigem Abstand untereinander auf einem Kreis symmetrisch zur Längsachse X-X des zylinderförmigen Gehäuses 31 angeordnet sind. Für den zylinderförmigen Innenbereich innerhalb des ringförmigen Strömungskanals 30 ist eine mittige Belüftungsbohrung 37 vorgesehen. Angesaugt wird im Bereich der Einsaugöffnung 34 in den Strömungskanal 30 durch ringsegmentförmige Einlässe 38, während die Haltescheibe 33 ringsegmentförmige Auslässe 39 besitzt. Eingesetzt sind die Haltescheiben 32 und 33 in einen im Durchmesser vergrößerten Innenabschnitt 40 bzw. 41 des zylinderförmigen Gehäuses 31 und liegen definiert an einer Schulter 42 bzw. 43 an.

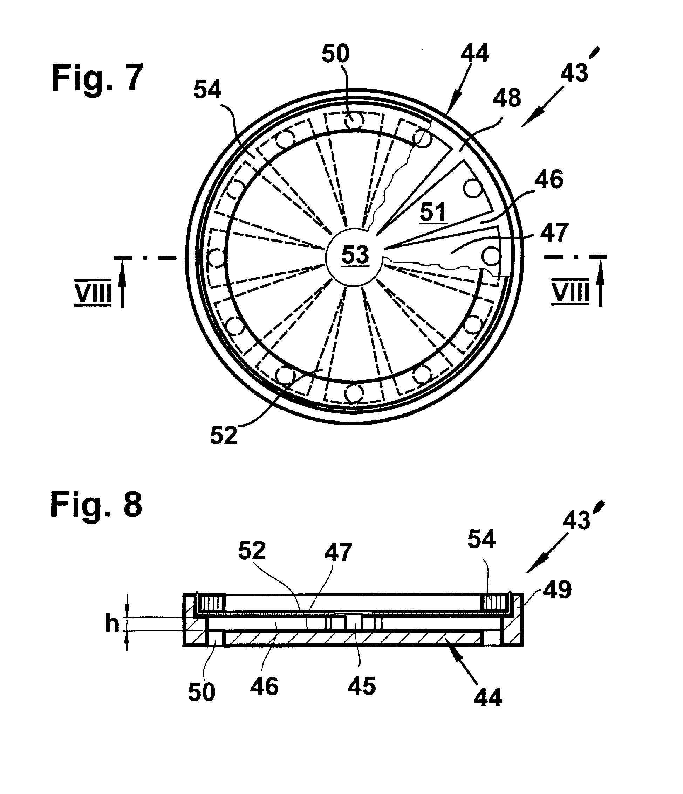

In Fig. 7 ist ein weiteres Ausführungsbeispiel einer erfindungsgemäßen Vorrichtung 43' zur Flussbegrenzung bei niedrigen Differenzdrücken dargestellt, die sich insbesondere für das Begrenzen des Inhalationsvolumenstroms bei der Inhalation von therapeutischen Aerosolen eignet. Die Vorrichtung 43' besteht aus einem scheibenförmigen Gehäuse 44, in dem, wie in Fig. 8 ersichtlich, eine scheibenförmige Ausnehmung 45 mit angeformten Stegen 46 gebildet ist. Die Stege 46 sind auf einem flachen Boden 47 der Ausnehmung 45 angeformt und erstrecken sich um eine Höhe h bis zu einem Absatz 48, der in dem Grundteil 44 gebildet ist. Von diesem Absatz 48, in den die Stege 46 glatt übergehen, erstreckt sich eine ringförmige Aufnahmeanformung 49 etwa um die gleiche Höhe h nach oben. Im ausgenommenen Teil des Grundteils 44 finden sich nahe des Außenrandes gleichmäßig radial verteilte Aussaugöffnungen 50, die am äußeren Ende jedes Strömungskanals 51 zwischen zwei benachbarten Stegen 46 angeordnet sind. Die Stege 46 sind bei dem Ausführungsbeispiel gemäß Fig. 7 gleich lang, radial von der Mitte des Gehäuses gesehen angeordnet und erweitern sich nach außen in ihrer Breite.

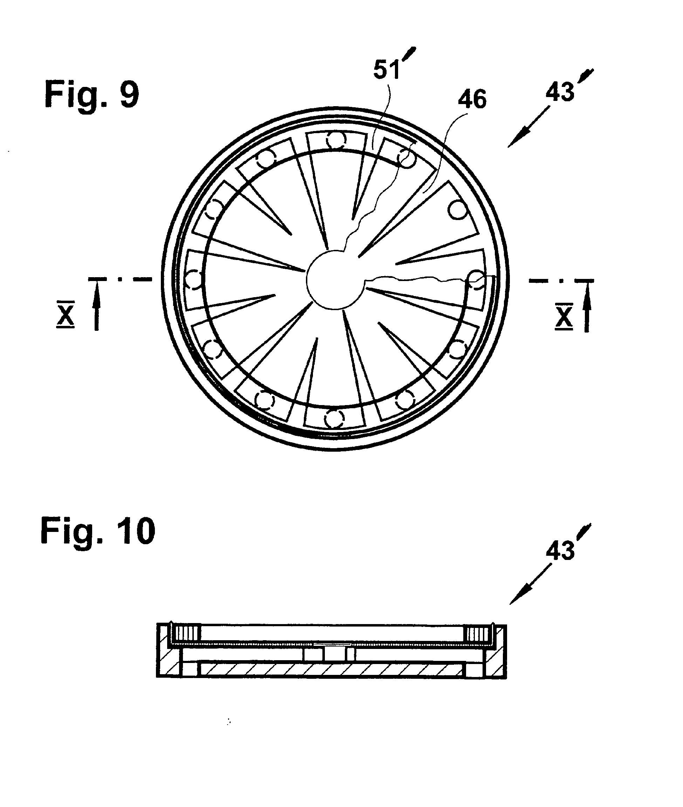

Bei dem Ausführungsbeispiel gemäß Fig. 9 liegen die Unterschiede darin, dass alternierend zwei verschiedene Stege 46 und 51 gleichmäßig voneinander beabstandet und zur Mitte spitz zulaufend vorgesehen sind, wodurch zunächst ein breiterer Strömungskanal im Zentrum des Gehäuses 44 gebildet ist, der dann radial nach außen in zwei Strömungskanäle aufgeteilt ist.

Auf dem kreisförmigen Gehäuse 44 ist eine dünne flexible Wand 52 derart angeordnet, dass sie auf den Stegen aufliegt und sich von einer mittigen Öffnung 53 in der Wand 52 nach außen über den Absatz 48 und im Bereich des Aufnahmeabschnitts 49 nach oben umgelegt erstreckt. Diese dünne flexible Wand 52 besteht beispielsweise aus Silikon und ist randseitig mittels eines Befestigungsringes 54 an dem Gehäuse 44 festgeklemmt. Der Befestigungsring 54 ist zum Austausch der Wand 52 aus seiner krafischlüssigen Befestigungsstellung lösbar und wieder einsetzbar.

Die in den Fig. 7 bis 10 dargestellten Ausführungsbeispiele der Vorrichtung 43' zur Flussbegrenzung arbeiten ebenso, wie die eingangs beschriebene Ausführung einer linearen Flussbegrenzung, bei der der Strömungskanal auch in mehrere Strömungskanäle 51 unterteilt werden kann, mit nur einer elastischen Wand 52 bzw. Matte. Das zuletzt beschriebene Ausfiihrungsbeispiel besitzt einen sternförmigen Aufbau mit mehreren sternförmig verlaufenden Strömungskanälen 51. Die Strömungskanäle 51 gehen dabei vom Zentrum aus und werden durch die flexible Wand 52 oberseitig, seitlich durch zwei sternförmig verlaufende Stege 46, 51' und an einer planaren Bedarfsfläche 47 gebildet. Dabei sind die Stege 46, 51' und die planare Bedarfsfläche 47 in einem Teil ausgebildet. Die Einsaugöffnung 53 ist durch eine sich bis zu den inneren Spitzen der Stege 46 erstreckende Öffnung in der flexiblen Wand 52 gebildet, während die Aussaugöffnungen 50 durch den unteren Teil des Gehäuses 44 im radialen Umfangsbereich gebildet sind.

Über die Höhe h der Stege 46 wird der Abstand der elastischen Wand 52 von der planaren Bedarfsfläche 47 festgelegt. Wird durch die Einsaugöffnung 53 angesaugt, entsteht aufgrund des Strömungswiderstandes ein Unterdruck in den durch die Strömungskanäle 51 gebildeten Strömungsbereichen. Gleichzeitig wird die dünne elastische Wand 52 durch diesen Unterdruck in die Strömungskanäle 51 hineingezogen und verursacht dadurch eine Verengung der Querschnitte der Strömungskanäle 51. Dabei nimmt die Durchbiegung der elastischen Wand 52 mit der Höhe des Unterdrucks in den Strömungskanälen 51 zu.

## Patentansprüche

1. Vorrichtung zur Flussbegrenzung bei niedrigen Differenzdrücken, insbesondere für das Begrenzen des Inhalationsvolumenstroms bei der Inhalation von therapeutischen Aerosolen, bestehend aus
einem Gehäuse (11, 31, 44) mit wenigstens einer Einsaugöffnung (14, 34, 53), wenigstens einer Aussaugöffnung (15, 35) und einem zwischen diesen angeordneten Strömungsbereich mit wenigstens einer flexiblen Wand (18, 20, 28, 39, 52), dessen Querschnitt abhängig von dem zwischen Aussaugöffnung (15, 35) und Einsaugöffnung (14) herrschenden Unterdruck und der Materialflexibilität des Wandmaterials bis zu einer vorbestimmten Größe für einen vorgegebenen maximalen Inhalationsvolumenstrom verringerbar ist,
**dadurch gekennzeichnet,**
**dass** der Strömungsbereich wenigstens einen Strömungskanal (23, 30) mit einem flachen länglichen Querschnitt aufweist.

2. Vorrichtung nach Anspruch 1,
**dadurch gekennzeichnet,**
**dass** der Querschnitt jedes Strömungskanals (23) des Strömungsbereichs mit gegenüberliegenden großflächigen Wänden (18, 20) gebildet ist.

3. Vorrichtung nach Anspruch 2, **dadurch gekennzeichnet, dass** die Wände (18, 20) an ihrer Außenseite wenigstens im Bereich der Mitte zwischen Einsaug- und Aussaugöffnung (14 bzw. 15) zur Umgebung offen (16, 17) sind.

4. Vorrichtung nach Anspruch 3,
**dadurch gekennzeichnet,**
**dass** jede Wand (18, 20) an ihrer Außenseite wenigstens im Bereich der Mitte zwischen der Einsaug- und Aussaugöffnung (14 bzw. 15) einen Kammerabschnitt (24, 25) aufweist, der über eine Belüftungsöffnung (16, 17) zur Umgebung geöffnet ist.

5. Vorrichtung nach einem der vorangehenden Ansprüche,
**dadurch gekennzeichnet,**
**dass** jeder Strömungskanal (23) des Strömungsbereichs einen schichtweisen Aufbau aufweist.

6. Vorrichtung nach Anspruch 5,
**dadurch gekennzeichnet,**
**dass** jeder Strömungskanal (23) des Strömungsbereichs aus einer geschlossenen Wand (20) einer gleich großen rahmenförmigen Zwischenwand (19) und einer gleich großen Wand (18) mit Einsaug- und Aussaugöffnung (21, 22) gebildet ist.

7. Vorrichtung nach einem der vorangehenden Ansprüche,
**dadurch gekennzeichnet,**
**dass** die gegenüberliegenden Wände (18, 20) jedes Strömungskanals (23) des Strömungsbereichs randseitig in dem Gehäuse (11) befestigt sind.

8. Vorrichtung nach einem der vorangehenden Ansprüche,
**dadurch gekennzeichnet,**
**dass** das flexible Material, mit dem jeder Strömungskanal (23) des Strömungsbereichs gebildet ist, aus bioverträglichem Kunststoff besteht.

9. Vorrichtung nach einem der vorangehenden Ansprüche,
**dadurch gekennzeichnet,**
**dass** wenigstens die Wände (18, 20) jedes Strömungskanals (23) des Strömungsbereichs aus Silikonmatten bestehen.

10. Vorrichtung nach einem der Ansprüche 5 bis 9,
**dadurch gekennzeichnet,**
**dass** die Materialschichten für jeden Strömungskanal (23) des Strömungsbereichs zwischen zwei Gehäuseabschnitten (12, 13) austauschbar befestigt sind.

11. Vorrichtung nach einem der vorangehenden Ansprüche,
**dadurch gekennzeichnet,**
**dass** die großflächigen Wände (18, 20) jedes Strömungskanals (23) des Strömungsbereichs die gleiche Wanddicke aufweisen.

12. Vorrichtung nach einem der Ansprüche 1 bis 4,
**dadurch gekennzeichnet,**
**dass** jeder Strömungskanal (23) des Strömungsbereichs einstückig aufgebaut ist.

13. Vorrichtung nach Anspruch 12,
**dadurch gekennzeichnet,**
**dass** jeder Strömungskanal (23) des Strömungsbereichs aus einem Silikonbauteil besteht.

14. Vorrichtung nach einem der Ansprüche 1 oder 2,
**dadurch gekennzeichnet,**
**dass** jede Wand (18, 20) an ihrer Außenseite wenigstens im Bereich der Mitte zwischen der Einsaug- und der Aussaugöffnung (14 bzw. 15) einen Kammerabschnitt (24, 25) mit einer Belüflungsbohrung (16, 17) aufweist, wobei die Belüftungsbohrungen (16, 17) über einen Kanal bzw. einen Schlauch mit der Einsaugöffrung (14) verbunden sind.

15. Vorrichtung zur Flussbegrentung bei niedrigen Differenzdrücken, insbesondere für das Begrenzen des Inhalationsvolumenstroms bei der Inhalation von therapeutischen Aerosolen, bestehend aus
einem Gehäuse (11, 31, 44) mit wenigstens einer Einsaugölhung (14, 34, 53), wenigstens einer Aussaugöffnung (15, 35) und einem zwischen diesen angeordneten Strömungsbereich mit wenigstens einer flexiblen Wand (18, 20, 28, 39, 52), dessen Querschnitt abhängig von dem zwischen Aussaugöffnung (15, 35) und Einsaugöffnung (14) herrschenden Unterdruck und der Materialflexibilität des Wandmaterials bis zu einer vorbestimmten Größe für einen vorgegebenen maximalen Inhalationsvolumenstrom verringerbar ist,
**dadurch gekennzeichnet,**
**dass** der Strömungsbereich wenigstens einen Strömungskanal (30) mit einem ringförmigen Querschnitt aufweist.

16. Vorrichtung nach Anspruch 15,
**dadurch gekennzeichnet,**
**dass** jeder Strömungskanal (30) des Strömungsbereichs in einem zylinderförmigen Gehäuse (31) symmetrisch und von der Zylinderinnenwand beabstandet zwischen radialen Haltescheiben (32, 33) angeordnet ist.

17. Vorrichtung nach Anspruch 16,
**dadurch gekennzeichnet,**
**dass** die Haltescheiben (32, 33) ringsegmentförmige Ein- und Auslässe (38, 39) für den Strömungskanal (30) des Strömungsbereichs aufweisen, wobei die die Einlässe (38) aufweisenden Haltescheiben (32) Belüftungsöffnungen (36, 37) für den zylinderförmigen Innenbereich und den ringförmigen Umgebungsbereich jedes Strömungskanals (30) aufweist.

18. Vorrichtung nach einem der Ansprüche 15 bis 17,
**dadurch gekennzeichnet,**
**dass** jeder ringförmige Strömungskanal (30) des Strömungsbereichs mit Silikonmatten(38, 39) gebildet ist.

19. Vorrichtung zur Flussbegrenzung bei niedrigen Differenzdrücken, insbesondere für das Begrenzen des Inhalationsvolumenstroms bei der Inhalation von therapeutischen Aerosolen, bestehend aus
einem Gehäuse (11, 31, 44) mit wenigstens einer Einsaugöffnung (14, 34, 53), wenigstens einer Aussaugöffnung (15, 35) und einem zwischen diesen angeordneten Strömungsbereich mit wenigstens einer flexiblen Wand (18, 20, 28, 39, 52), dessen Querschnitt abhängig von dem zwischen Aussaugöffnung (15, 35) und Einsaugöffnung (14) herrschenden Unterdruck und der Materialflexibilität des Wandmaterials bis zu einer vorbestimmten Größe für einen vorgegebenen maximalen Inhalationsvolumenstrom verringerbar ist,
**dadurch gekennzeichnet,**
**dass** der Strömungsbereich (51) zwischen einer zentralen Einsaugöffnung (53) und diese radial umgebenden Aussaugöffnungen (50) gebildet ist und sternförmige Stege (46, 51) aufweist, die sich von einer gemeinsamen Bodenfläche (47) zu der flexiblen Wand (52) erstrecken und Strömungskanäle bilden.

20. Vorrichtung nach Anspruch 19,
**dadurch gekennzeichnet,**
**dass** die Stege (46, 51') unterschiedliche Längen aufweisen.

21. Vorrichtung nach Anspruch 19 oder 20,
**dadurch gekennzeichnet,**
**dass** sich die Stege (46, 51') in ihrer Breite nach außen erweitern.

22. Vorrichtung nach einem der Ansprüche 19 bis 21,
**dadurch gekennzeichnet,**
**dass** zwischen zwei benachbarten Stegen (46, 51') wenigstens eine Aussaugöffnung (50) vorgesehen ist.

23. Vorrichtung nach einem der Ansprüche 19 bis 22,
**gekennzeichnet durch**
ein scheibenförmiges Gehäuse (44), in dem einstückig zwischen flachen Ausnehmungen (47) die Stege (46, 51') und randseitig die Aussaugöffnungen (50) gebildet sind, wobei eine dünne flexible Wand (52) mit mittiger Einsaugöffnung (53) auf den Stegen (46, 51) aufliegt und im Randbereich des Gehäuses (44) befestigt ist.

24. Vorrichtung nach Anspruch 23,
**dadurch gekennzeichnet,**
**dass** die Wand (52) im Randbereich des Gehäuses (44) festgeklebt bzw. festgeschweißt oder mittels eines ringförmigen Montageteils (54) festgeklemmt ist.

25. Vorrichtung nach Anspruch 23 oder 24,
**dadurch gekennzeichnet,**
**dass** die flexible Wand (52) aus Silikon, Silikonkautschuk, Viton, Latex, Naturkautschuk oder anderen Elastomeren besteht.

26. Vorrichtung nach einem der vorangehenden Amprüche,
**dadurch gekennzeichnet,**
**dass** die Wände (18, 20, 38, 39, 52) eine degressive Materialflexibilität aufweisen.

## Claims

1. Device for flow rate limitation at low differential pressures, particularly for limiting the volumetric inhalation flow during inhalation of therapeutic aerosols, consisting of
a housing (11, 31, 44) with at least one aspiration orifice (14, 34, 53), at least one inhalation orifice (15, 35) and a flow space disposed therebetween and including at least one flexible wall (18, 20, 28, 39, 52), having a cross-section which can be reduced as a function of the differential pressure prevailing between said inhalation orifice (15, 35) and said aspiration orifice (14) and of the flexibility of the wall material up to a predetermined dimension for an definable maximum volumetric inhalation flow,
**characterized in**
**that** said flow space comprises at least one flow passage (23, 30) having a flat elongate cross-section.

2. Device according to Claim 1,
**characterized in**
**that** the cross-section of each flow passage (23) of said flow space is formed to have opposing large-area walls (18, 20).

3. Device according to Claim 2,
**characterized in**
**that** said walls (18, 20) are open (16, 17) on their outside at least in the central region between the aspiration an inhalation orifices (14 or 15, respectively) towards the outside environment.

4. Device according to Claim 3,
**characterized in**
**that** each wall (18, 20) includes a chamber section (24, 25) on its outside at least in the central region between said aspiration an inhalation orifices (14 or 15, respectively), which section is open towards the environment through a bore (16, 17).

5. Device according to any of the preceding Claims,
**characterized in**
**that** each flow passage (23) of said flow space has a stratified structure.

6. Device according to Claim 5,
**characterized in**
**that** each flow passage (23) of said flow space is formed by a closed wall (20) of a frame-shaped partition (19) of equal size and a wall (18) of the same size with aspiration and inhalation orifices (21, 22).

7. Device according to any of the preceding Claims,
**characterized in**
**that** said opposing walls (18, 20) of each flow passage (23) of said flow space are fastened on the edge side in said housing (11).

8. Device according to any of the preceding Claims,
**characterized in**
**that** the flexible material used to form each flow passage (23) of said flow space consists of a biological tolerable synthetic material.

9. Device according to any of the preceding Claims,
**characterized in**
**that** at least said large-area passage walls (18, 20) of each flow passage (23) of said flow space consist of silicone mats.

10. Device according to any of the Claims 5 to 9,
**characterized in**
**that** the material layers for each flow passage (23) of said flow space are fastened for exchange between two housing sections (12, 13).

11. Device according to any of the preceding Claims,
**characterized in**
**that** said large-area walls (18, 20) of each flow passage (23) of said flow space have the same wall thickness.

12. Device according to any of the Claims 1 to 4,
**characterized in**
**that** each flow passage (23) of said flow space has an integral structure.

13. Device according to Claim 12,
**characterized in**
**that** each flow passage (23) of said flow space consists of a silicone component.

14. Device according to any of the Claims 1 or 2,
**characterized in**
**that** each wall (18, 20) comprises a chamber section (24, 25) on its outside at least in the central region between said aspiration and inhalation orifices (14) or (15), respectively, which section includes a bore (16, 17), said bores (16, 17) communicating with said aspiration orifice (14) via a passage or a hose, respectively.

15. Device for flow rate limitation at low differential pressures, particularly for limiting the volumetric inhalation flow during inhalation of therapeutic aerosols, consisting of
a housing (11, 31, 44) with at least one aspiration orifice (14, 34, 53), at least one inhalation orifice (15, 35) and a flow space disposed therebetween and including at least one flexible wall (18, 20, 28, 39, 52), having a cross-section which can be reduced as a function of the differential pressure prevailing between said inhalation orifice (15, 35) and said aspiration orifice (14) and of the flexibility of the wall material up to a predetermined dimension for an definable maximum volumetric inhalation flow,
**characterized in**
**that** said flow space comprises at least one flow passage (30) having an annular cross-section.

16. Device according to Claim 15,
**characterized in**
**that** each flow passage (30) of said flow space is symmetrically disposed in a cylindrical housing (31) at a spacing from the inner wall of the cylinder, between radial retainer disks (32, 33).

17. Device according to Claim 16,
**characterized in**
**that** said retainer disks (32, 33) comprise inlets and outlets (38, 39) having the shape of ring segments, for the flow passage (30) of said flow space, with said retainer disks (32) including said inlets (38) comprising pressure-equalizing bores (36, 37) for said cylindrical interior space and the annular zone surrounding each flow passage (30) .

18. Device according to any of the Claims 15 to 17,
**characterized in**
**that** each annular flow passage (30) of said flow space is formed by silicone mats (38, 39).

19. Device for flow rate limitation at low differential pressures, particularly for limiting the volumetric inhalation flow during inhalation of therapeutic aerosols, consisting of
a housing (11, 31, 44) with at least one aspiration orifice (14, 34, 53), at least one inhalation orifice (15, 35) and a flow space disposed therebetween and including at least one flexible wall (18, 20, 28, 39, 52), having a cross-section which can be reduced as a function of the differential pressure prevailing between said inhalation orifice (15, 35) and said aspiration orifice (14) and of the flexibility of the wall material up to a predetermined dimension for an definable maximum volumetric inhalation flow,
**characterized in**
**that** the flow space (51) is formed between a central aspiration orifice (53) and inhalation orifices (50) surrounding radially the aspiration orifice (53), and comprises radial webs (46, 51') extending from a common bottom surface (47) to the flexible wall (52) and forming flow passages.

20. Device according to Claim 19,
**characterized in**
**that** said webs (46, 51') have different lengths.

21. Device according to Claim 19 or 20,
**characterized in**
**that** each web (46, 51') has an increased width outwards.

22. Device according to any of the Claims 19 to 21,
**characterized in**
**that** at least one inhalation orifice (50) is provided between two adjacent webs (46, 51').

23. Device according to any of the Claims 19 to 22,
**characterized by**
a disk-shaped housing (44), in which said webs (46, 51') are integrally formed between flat recesses (47) and said inhalation orifices (50) are formed on the edge side, wherein a thin flexible wall (52) with a central aspiration orifice (53) is supported by said webs (46, 51') and fastened on the edge side of said housing (44).

24. Device according to Claim 23,
**characterized in**
**that** the wall (52) is joined with adhesives or welded, respectively, or clamped by means of an annular fixing element (54) on the edge side of said housing (44).

25. Device according to Claim 23 or 24,
**characterized in**
**that** the flexible wall (52) consists of silicone, silicone gum, Viton, Latex, natural rubber or other elastomer materials.

26. Device according to any of the preceding Claims,
**characterized in**
**that** the walls (18, 20, 38, 39, 52) have a declining material flexibility.

## Revendications

1. Dispositif pour limiter un écoulement lors de pressions différentielles basses, en particulier pour limiter le débit volumétrique d'inhalation lors de l'inhalation d'aérosols thérapeutiques, comprenant
un boîtier (11, 31, 44) avec au moins une ouverture d'entrée (14, 34, 53), au moins une ouverture de sortie (15, 35), et une zone d'écoulement disposée entre les deux ayant au moins une paroi flexible (18, 20, 28, 39, 52) dont la coupe transversale peut être diminuée jusqu'à une taille prédéterminée pour un débit volumétrique d'inhalation prédéterminé maximal, en fonction de la dépression régnante entre l'ouverture de sortie (15, 35) et l'ouverture d'entrée (14) et de la flexibilité du matériau de la
paroi,
**caractérisé en ce que**
la zone d'écoulement comprend au moins un canal d'écoulement (23, 30) avec une coupe transversale allongée plane.

2. Dispositif selon la revendication 1,
**caractérisé en ce que**
la coupe transversale de chaque canal d'écoulement (23) de la zone d'écoulement est formée avec des parois (18, 20) à grande surface se faisant face.

3. Dispositif selon la revendication 2,
**caractérisé en ce que**
les parois (18, 20) sont ouvertes (16, 17) vers l'extérieur au niveau de leurs faces externes au moins dans la zone du milieu entre l'ouverture d'entrée et de sortie (14 respectivement 15).

4. Dispositif selon la revendication 3,
**caractérisé en ce que**
chaque paroi (18, 20) comprend une partie chambre (24, 25) au niveau sa face externe au moins dans la zone du milieu entre l'ouverture d'entrée et de sortie (14 respectivement 15), celle-ci étant ouverte vers l'extérieur via une ouverture d'aération (16, 17).

5. Dispositif selon l'une quelconque des revendications précédentes,
**caractérisé en ce que**
chaque canal d'écoulement (23) de la zone d'écoulement comporte une structure en couches.

6. Dispositif selon la revendication 5,
**caractérisé en ce que**
chaque canal d'écoulement (23) de la zone d'écoulement est formé à partir d'une paroi fermée (20), d'une paroi intermédiaire (19)
identiquement grande en forme de cadre et d'une paroi (18) identiquement grande avec ouvertures d'entrée et de sortie (21, 22).

7. Dispositif selon l'une quelconque des revendications précédentes,
**caractérisé en ce que**
les parois se faisant face (18 ,20) de chaque canal d'écoulement (23) de la zone d'écoulement sont fixées à la périphérie du boîtier (11).

8. Dispositif selon l'une quelconque des revendications précédentes,
**caractérisé en ce que**
le matériau flexible, avec lequel chaque canal d'écoulement (23) de la zone d'écoulement est formé, est en matière plastique biocompatible.

9. Dispositif selon l'une quelconque des revendications précédentes,
**caractérisé en ce qu'**
au moins les parois (18, 20) de chaque canal d'écoulement (23) de la zone d'écoulement sont en nattes de silicone.

10. Dispositif selon une des revendications 5 à 9,
**caractérisé en ce que**
les couches de matériau pour chaque canal d'écoulement (23) de la zone d'écoulement sont fixées de façon échangeable entre deux parties de boîtier (12, 13).

11. Dispositif selon l'une quelconque des revendications précédentes,
**caractérisé en ce que**
les parois à grande surface (18, 20) de chaque canal d'écoulement (23) de la zone d'écoulement présentent la même épaisseur de paroi.

12. Dispositif selon une des revendications 1 à 4,
**caractérisé en ce que**
chaque canal d'écoulement (23) de la zone d'écoulement est construit en une seule pièce.

13. Dispositif selon la revendication 12,
**caractérisé en ce que**
chaque canal d'écoulement (23) de la zone d'écoulement est constitué en un composant silicone.

14. Dispositif selon une des revendications 1 ou 2,
**caractérisé en ce que**
chaque paroi (18, 20) comprend, au niveau de sa face externe au moins dans la zone du milieu entre l'ouverture d'entrée et de sortie (14 respectivement 15), une partie chambre (24, 25) avec un trou d'aération (16, 17), les trous d'aération (16, 17) étant reliés via un canal ou un tuyau avec l'ouverture d'entrée (14).

15. Dispositif pour limiter un écoulement lors de pressions différentielles basses, en particulier pour limiter le débit volumétrique d'inhalation lors de l'inhalation d'aérosols thérapeutiques, comprenant
un boîtier (11, 31, 44) avec au moins une ouverture d'entrée (14, 34, 53), au moins une ouverture de sortie (15, 35), et une zone d'écoulement disposée entre les deux ayant au moins une paroi flexible (18, 20, 28, 39, 52) dont la coupe transversale peut être diminuée jusqu'à une taille prédéterminée pour un débit volumétrique d'inhalation prédéterminé maximal, en fonction de la dépression régnante entre l'ouverture de sortie (15, 35) et l'ouverture d'entrée (14) et de la flexibilité du matériau de la
paroi,
**caractérisé en ce que**
la zone d'écoulement comprend au moins un canal d'écoulement (30) avec une coupe transversale en forme d'anneau.

16. Dispositif selon la revendication 15,
**caractérisé en ce que**
chaque canal d'écoulement (30) de la zone d'écoulement est disposé dans un boîtier de forme cylindrique (31), entre des disques de maintien (32, 33) radiaux, de façon symétrique et espacée de la paroi intérieure du cylindre.

17. Dispositif selon la revendication 16,
**caractérisé en ce que**
les disques de maintien (32, 33) comprennent des entrées et des sorties (38, 39) en forme de segment d'anneau pour le canal d'écoulement (30) de la zone d'écoulement, les disques de maintien (32) qui comprennent les entrées (38) comportant des ouvertures d'aération (36, 37) pour la zone intérieure, en forme de cylindre, et la zone extérieure, en forme d'anneau, de chaque canal d'écoulement (30).

18. Dispositif selon une des revendications 15 à 17,
**caractérisé en ce que**
chaque canal d'écoulement (30), en forme d'anneau, de la zone d'écoulement est formé avec des nattes de silicone (38, 39).

19. Dispositif pour limiter un écoulement lors de pressions différentielles basses, en particulier pour limiter le débit volumétrique d'inhalation lors de l'inhalation d'aérosols thérapeutiques, comprenant
un boîtier (11, 31, 44) avec au moins une ouverture d'entrée (14, 34, 53), au moins une ouverture de sortie (15, 35), et une zone d'écoulement disposée entre les deux ayant au moins une paroi flexible (18, 20, 28, 39, 52) dont la coupe transversale peut être diminuée jusqu'à une taille prédéterminée pour un débit volumétrique d'inhalation prédéterminé maximal, en fonction de la dépression régnante entre l'ouverture de sortie (15, 35) et l'ouverture d'entrée (14) et de la flexibilité du matériau de la paroi,
**caractérisé en ce que**
la zone d'écoulement (51) est formée entre une ouverture d'entrée (53) centrale et des ouvertures de sortie (50) l'entourant radialement et comprend des traverses (46, 51') en forme d'étoile qui se projettent d'une surface de fond commune (47) jusqu'à la paroi flexible (52) et forment des canaux d'écoulement.

20. Dispositif selon la revendication 19,
**caractérisé en ce que**
les traverses (46, 51') présentent des longueurs différentes.

21. Dispositif selon la revendication 19 ou 20,
**caractérisé en ce que**
les traverses (46, 51') s'élargissent dans la largeur vers l'extérieur.

22. Dispositif selon une des revendications 19 à 21,
**caractérisé en ce que**
au moins une ouverture de sortie (50) est prévue entre deux traverses (46, 51') voisines.

23. Dispositif selon une des revendications 19 à 22,
**caractérisé par**
un boîtier en forme de disque (44) dans lequel les traverses (46, 51') sont formées en une seule pièce entre des évidements planes (47) et les ouvertures de sortie (50) sont formées à la périphérie,
une paroi flexible fine (52) avec ouverture d'entrée centrale (53) est placée sur les traverses (46, 51) et est fixée à la périphérie du boîtier (44).

24. Dispositif selon la revendication 23,
**caractérisé en ce que**
la paroi (52) est collée ou soudée ou serrée à l'aide d'une pièce d'assemblage (54) en forme d'anneau à la périphérie du boîtier (44).

25. Dispositif selon la revendication 23 ou 24,
**caractérisé en ce que**
la paroi flexible (52) est en silicone, caoutchouc silicone, Viton, latex, caoutchouc naturel ou autres élastomères.

26. Dispositif selon l'une quelconque des revendications précédentes
**caractérisé en ce que**
les parois (18, 20, 38, 39, 52) présentent une flexibilité de matériau dégressive.
